# EUROPEAN PATENT SPECIFICATION

(11) **EP 0 473 229 B1**
(45) Date of publication and mention of the grant of the patent: **15.11.1995**
(21) Application number: 91202144.1
(22) Date of filing: 22.08.1991
(51) Int. Cl.: C11D 3/39

(54) **Bleaching and detergent compositions**
Bleich- und Waschmittel
Compositions détergentes pour blanchiment

(30) Priority: 28.08.1990 GB 9018749
(43) Date of publication of application: 04.03.1992
(73) Proprietor: UNILEVER N.V., 3013 AL Rotterdam (NL); UNILEVER PLC, London EC4P 4BQ (GB)
(72) Inventor: Thornthwaite, David William, Unilever Research, Bebington, Wirral Merseyside L63 3JW (GB); Oakes, John, Unilever Research, Bebington, Wirral Merseyside L63 3JW (GB); Kerr, Colin Watt, Unilever Research, Bebington, Wirral Merseyside L63 3JW (GB); Cotter, Byron R.,, Edgewater, New Jersey 07020 (US)
(74) Representative: Bryant, Tracey

(56) References cited:
- EP-A- 0 290 292
- EP-A- 0 399 584

## Description

### Field of the Invention

This invention relates to compositions suitable for the effective use in fabric washing and bleaching operations over a wide range of temperatures, especially at the lower temperature region.

### Background of the Invention

Detergent bleach compositions for washing at higher temperatures are well known in the art. Normally, as bleaching agent, they contain a peroxide compound which liberates hydrogen peroxide in aqueous solution, such as peroxyhydrates, sodium perborate mono- or tetra-hydrate, sodium percarbonate, sodium perpyrophosphate, sodium persilicate, urea peroxide and the like. These bleaching agents are only effective at higher temperatures, i.e. from 80°C up to the boil.

Organic peroxyacids, as a class, are very effective bleaches and the use thereof as the bleach system in detergent compositions has been proposed in the art. Although the efficacy of certain organic peroxyacid compounds at low to medium wash temperatures has long been recognized, the major problem, which has prevented their commercial exploitation so far, is their inherent instability in conventional alkaline fabric washing detergent compositions. Unprotected or insufficiently protected peroxyacid compounds will also attack sensitive ingredients, such as fluorescers, enzymes and perfumes, and can cause severe pin-point spotting during use upon fabrics.

Because of these and other problems connected with peroxyacids **per se**, there exists a continuing interest in the use of bleach systems which generate the organic peroxyacid **in situ**, i.e. in the form of a peroxyacid bleach precursor.

Numerous substances have been disclosed and proposed in the art as usable peroxyacid bleach precursors. Conventionally, these precursors are reactive organic compounds having an O-acyl or N-acyl group, such as the carboxylic esters, that in alkaline solutions containing a source of hydrogen peroxide, e.g. perborate, will generate the corresponding peroxyacids, a reaction which is also referred to as perhydrolysis. Conventional precursors can be represented by the following general formula : 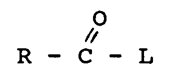
wherein R can be any suitable radical forming the RCO (acyl) group and L is a suitable leaving group. It is believed that the reaction with hydrogen peroxide proceeds as follows : 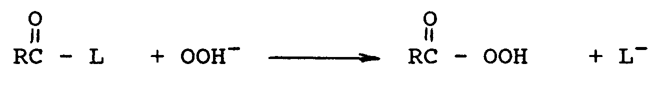
A leaving group is thus any group that is displaced from the peroxyacid bleach precursor as a consequence of nucleophilic attack on the precursor by the hydroperoxide anion. This, i.e. the perhydrolysis reaction, results in the formation of the peroxyacid. Generally, for a group to be a suitable leaving group, it must exert an electron-attracting effect, which facilitates expulsion of the leaving group from the tetrahydral intermediate formed by nucleophilic attack by the hydroperoxide anion. Many and diverse leaving group structures have been described in the patent literature (see, for example, EP-A-0120591). Not only do leaving groups add extra weight to bleach precursors of the conventional type but, once expelled from the precursor as a consequence of nucleophilic attack, they will remain as substantially useless by-products in the bleach solution.

As 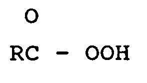
is the peroxyacid formed, the type thereof is entirely determined by the radical R.

Examples of the most representative precursors of this broad class include N,N,N',N'-tetraacetyl ethylene diamine (TAED), glucose pentaacetate (GPA), xylose tetraacetate (TAX), sodium-4-benzoyloxy benzene sulphonate (SBOBS), sodiumtrimethyl hexanoyloxy benzene sulphonate (STHOBS), tetraacetyl glucoluril (TAGU), tetraacetyl cyanuric acid (TACA), di-N-acetyldimethyl glyoxine (ADMG) and 1-phenyl-3-acetylhydantoin (PAH) - see, for example, GB-A-836,988; GB-A-907,356; EP-A-0098129 and EP-A-0120591, which represent only a small part of the large amount of patent literature disclosing precursors.

### Description of the Invention

The present invention now provides a novel peroxyacid bleach precursor compound having the following general formula : 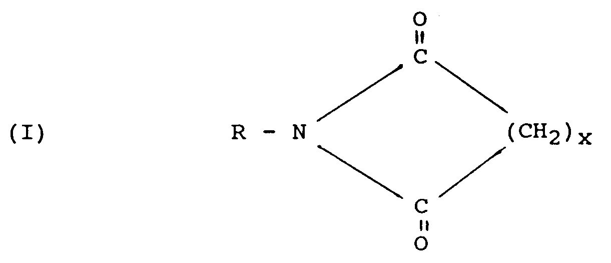
wherein R is an alkyl, aryl or alkaryl group containing from 1 to 10 carbon atoms, or a quaternary ammonium-substituted alkyl, aryl or alkaryl group containing from 1 to 10 carbon atoms; and x is an integer from 2 to 6.

Preferably, R is an alkyl group containing 1 to 4 carbon atoms, or a phenyl (optionally substituted) group, or a trimethyl ammonium C₂-C₄ alkylene or phenylene group; and x is preferably 2-4.

A particularly preferred bleach precursor is that wherein R is quaternary ammonium-substituted, more particularly a trimethyl ammonium alkylene or phenylene group.

The novel bleach precursors of the invention are thus cyclic imides which perhydrolyse with the formation of peroxyacids containing an internal amide linkage of the following general formula : 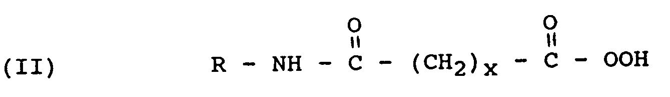
Without wishing to be bound by any theory, it is believed that the perhydrolysis reaction proceeds via ring cleavage at one of the N-acyl bonds, as diagrammatically shown below : 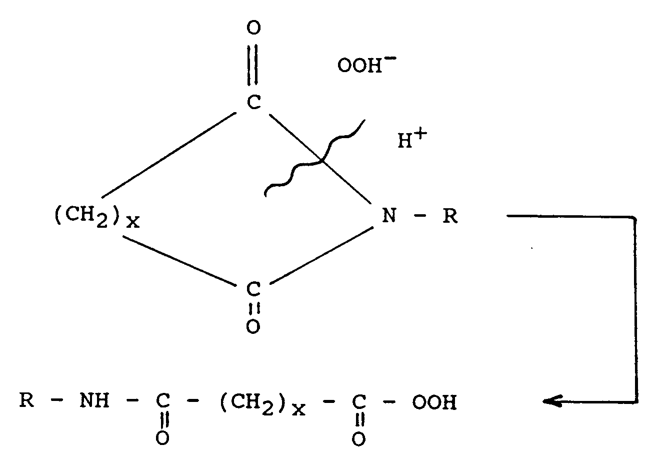
Some peroxyacids of this class are known from EP-A-0170386 and EP-A-0290292. They are effective over a wide range of temperatures. These peroxyacids are generated **in situ** according to the invention, forming the effective bleaching species.

An advantage of the bleach precursors according to the invention over the bleach precursors of the art is that they do not contain a leaving group. The bleach precursors of the invention which, upon perhydrolysis, produce the neat amido-peroxyacids directly, without by-products originating from the leaving group, are thus more weight-effective and chemically pure.

When R is a quaternary ammonium-substituted group, the compound is a cationic peroxyacid bleach precursor, which will generate the corresponding quaternary ammonium-substituted amido-peroxyacids which, because of their cationic nature, are substantive and effective bleaches but which also, because of their internal amide linkage, will be thermally more stable than the known cationic peroxyacids of the art.

Such cyclic imide compound having a quaternary ammonium substituent is, for example : 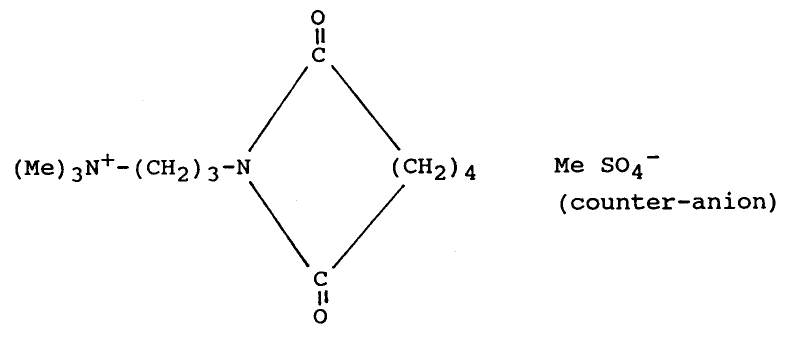
which, upon perhydrolysis, will yield a cationic amido-peroxyacid of the following formula : 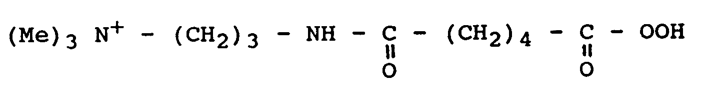
It should be appreciated that MeSO₄⁻ is used above as the counter-ion by way of example and that it is possible, in principle, to use any other known anion, such as, for example, Cl⁻, Br⁻ or NO₃⁻, as counter-anion for the cationic quaternary ammonium group.

The cyclic imide compounds usable as peroxyacid bleach precursors of the invention can be synthesized from reacting an amine with a dicarboxylic acid anhydride, followed by cylization of the amino-oxo-acid according to the general scheme given below : 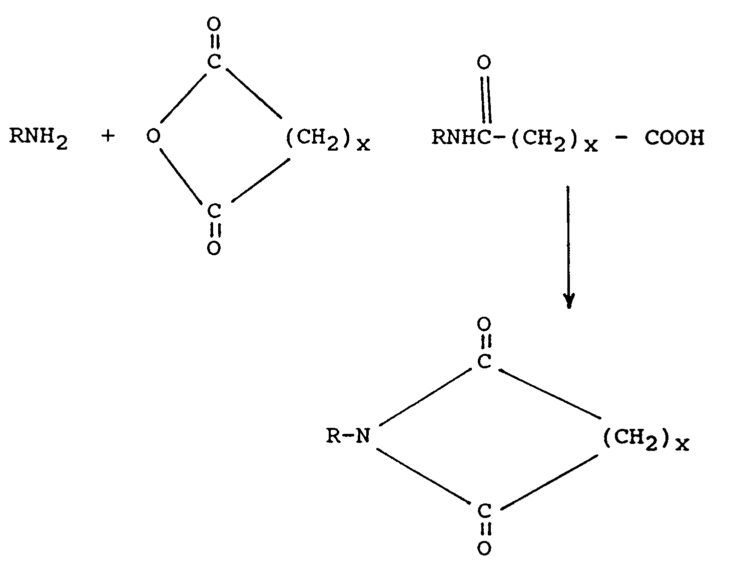
The invention also relates to bleaching compositions comprising one or more of the above bleach precursor compounds and a peroxide compound capable of yielding hydrogen peroxide in aqueous solution. In a preferred embodiment the bleaching compositions are incorporated in detergent compositions.

In such compositions the cyclic imide peroxyacid bleach precursor can be used with hydrogen peroxide or a hydrogen peroxide source in the form of a solid peroxide compound, such as sodium perborate or sodium percarbonate, in molar ratios of hydrogen peroxide to precursor of at least 1:1, preferably from about 2:1 to about 20:1, more preferably from 5:1 to 12:1.

The bleaching compositions of the invention provide extremely effective and efficient surface bleaching of textiles and fabrics over a wide temperature range from about 10°C to about 85°C, preferably at temperatures ranging from 20°C to 60°C, particularly preferably from 30°C to 50°C, thereby removing stains and/or soils from the textiles and fabrics. The compositions are particularly effective at removing dingy soils.

Surface bleaching performance is obtained with bleaching solutions already at pH 7.5, though each precursor compound appears to have its optimum pH ranging from 8.5 to about 11.0. Such pH can be obtained with substances commonly known as buffering agents, which are optional components of the bleaching composition herein.

The bleach precursors within the invention can also render peroxide bleaches more efficient, even at bleach solution temperatures wherein bleach activators are not necessary to activate the bleach, i.e. above about 60°C. Therefore, with bleach compositions of the invention, less peroxide bleach is required to get the same level of surface-bleaching performance as is obtained with the peroxide bleach alone.

### The Peroxide Bleaching Compound

The peroxide bleaching compounds useful herein are those capable of yielding hydrogen peroxide in an aqueous solution. These compounds are well known in the art and include hydrogen peroxide and the alkali metal peroxides, organic peroxide bleaching compounds, such as urea peroxide, and inorganic persalt bleaching compounds, such as the alkali metal perborates, percarbonates, perphosphates, and the like. Mixtures of two or more such bleaching compounds can also be used, if desired.

Preferred peroxide bleaching compounds include sodium perborate, commercially available in the form of mono-, tri- and tetra-hydrate, sodium carbonate peroxyhydrate, sodium pyrophosphate peroxyhydrate, urea peroxyhydrate, and sodium peroxide. Particularly preferred are sodium perborate tetrahydrate and, especially, sodium perborate monohydrate. Sodium perborate monohydrate is especially preferred because it is very stable during storage and yet still dissolves very quickly in the bleaching solution. It is believed that such rapid dissolution results in the formation of higher levels of percarboxylic acid and, thus, enhanced surface-bleaching performance.

The level of bleach precursor within the compositions of the invention is from about 0.1% to about 60% and preferably from about 0.5% to about 40% by weight. When the bleaching compositions within the invention are also detergent compositions, it is preferred that the level of bleach precursor is from about 0.5% to about 20% by weight, particularly from 1% to 10% by weight.

In these compositions the level of peroxide compound bleach that can be used is generally from 2% to 80%, preferably from 4% to 30%, particularly from 10 to 25% by weight.

### Optional Components

As a preferred embodiment, the bleaching compositions of the invention can be detergent compositions. Thus, the bleaching compositions can contain typical detergent composition components such as detergency surfactants and detergency builders. In such preferred embodiments the bleaching compositions are particularly effective. The bleaching compositions of this invention can contain all of the usual components of detergent compositions including the ingredients set forth in US Patent 3,936,537. Such components include colour speckles, suds boosters, suds suppressors, anti-tarnish and/or anti-corrosion agents, soil-suspending agents, soil-release agents, dyes, fillers, optical brighteners, germicides, alkalinity sources, hydrotropes, anti-oxidants, enzymes, enzyme-stabilizing agents, perfumes, etc.

Enzymes are highly preferred optional ingredients and are incorporated in an amount of from about 0.025% to about 5%, preferably from about 0.05% to about 1.5% by weight. A proteolytic activity of from about 0.01 to about 0.05 Anson units per gram of product is desirable. Other enzymes, including amylolytic enzymes, are also desirably included in the present compositions.

Suitable proteolytic enzymes include the many species known to be adapted for use in detergent compositions. Commercial enzyme preparations such as "Alcalase" sold by Novo Industries, and "Maxatase" sold by Gist-Brocades, Delft, The Netherlands, are suitable. Other preferred enzyme compositions include those commercially available under the trade-names SP-72 ("Esperase") manufactured and sold by Novo Industries A/S, Copenhagen, Denmark, and "AZ-Protease" manufactured and sold by Gist-Brocades, Delft, The Netherlands.

Suitable amylases include "Rapidase" sold by Gist-Brocades and "Termamyl" sold by Novo Industries.

A more complete disclosure of suitable enzymes can be found in US Patent 4,101,457.

The detergent surfactants can be any one or more surface-active agents selected from anionic, nonionic, zwitterionic, amphoteric and cationic classes and compatible mixtures thereof. Detergent surfactants useful herein are listed in US Patent 3,664,961 and US Patent 3,919,678. Useful cationic surfactants also include those described in US Patent 4,222,905 and US Patent 4,239,659. The following are representative examples of detergent surfactants useful in the present compositions.

Water-soluble salts of the higher fatty acids, i.e. "soaps", are useful anionic surfactants in the compositions herein. This includes alkali metal soaps such as the sodium, potassium, ammonium, and alkylol ammonium salts of higher fatty acids containing from about 8 to about 24 carbon atoms, and preferably from about 12 to about 18 carbon atoms. Soaps can be made by direct saponification of fats and oils or by the neutralization of free fatty acids. Particularly useful are the sodium and potassium salts of the mixtures of fatty acids derived from coconut oil and tallow, i.e. sodium or potassium tallow and coconut soap.

Useful anionic surfactants also include the water-soluble salts, preferably the alkali metal, ammonium and alkylol ammonium salts, of organic sulphuric reaction products having in their molecular structure an alkyl group containing from about 10 to about 20 carbon atoms and a sulphonic acid or sulphuric acid ester group. (Included in the term "alkyl" is the alkyl portion of acyl groups.) Examples of this group of synthetic surfactants are the sodium and potassium alkyl sulphates, especially those obtained by sulphating the higher alcohols (C₈-C₁₈ carbon atoms) such as those produced by reducing the glycerides of tallow or coconut oil; and the sodium and potassium alkyl benzene sulphonates in which the alkyl group contains from about 9 to about 15 carbon atoms, in straight chain or branched chain configuration, e.g., those of the type described in US Patents 2,220,099 and 2,477,383. Especially valuable are linear straight chain alkyl benzene sulphonates in which the average number of carbon atoms in the alkyl group is from about 11 to 13, abbreviated as C₁₁₋₁₃LAS.

Other anionic surfactants herein are the sodium alkyl glyceryl ether sulphonates, especially those ethers of higher alcohols derived from tallow and coconut oil; sodium coconut oil fatty acid monoglyceride sulphonates and sulphates; sodium or potassium salts of alkyl phenol ethylene oxide ether sulphates containing from about 1 to about 10 units of ethylene oxide per molecule and wherein the alkyl groups contain from about 8 to about 12 carbon atoms; and sodium or potassium salts of alkyl ethylene oxide ether sulphates containing about 1 to about 10 units of ethylene oxide per molecule and wherein the alkyl group contains from about 10 to about 20 carbon atoms.

Other useful anionic surfactants herein include the water-soluble salts of esters of alpha-sulphonated fatty acids containing from about 6 to 20 carbon atoms in the fatty acid group and from about 1 to 10 carbon atoms in the ester group; water-soluble salts of 2-acyloxyalkane-1-sulphonic acids containing from about 2 to 9 carbon atoms in the acyl group and from about 9 to about 23 carbon atoms in the alkane moiety; water-soluble salts of olein and paraffin sulphonates containing from about 12 to 20 carbon atoms; and beta-alkyloxy alkane sulphonates containing from about 1 to 3 carbon atoms in the alkyl group and from about 8 to 20 carbon atoms in the alkane moiety.

Water-soluble nonionic surfactants are also useful in the compositions of the invention. Such nonionic materials include compounds produced by the condensation of alkylene oxide groups (hydrophilic in nature) with an organic hydrophobic compound, which may be aliphatic or alkyl aromatic in nature. The length of the polyoxyalkylene group which is condensed with any particular hydrophobic group can be readily adjusted to yield a water-soluble compound having the desired degree of balance between hydrophilic and hydrophobic elements.

Suitable nonionic surfactants include the polyethylene oxide condensates of alkyl phenols, e.g. the condensation products of alkyl phenols having an alkyl group containing from about 6 to 15 carbon atoms, in either a straight chain or branched chain configuration, with from about 3 to 12 moles of ethylene oxide per mole of alkyl phenol.

Preferred nonionics are the water-soluble and water-dispersible condensation products of aliphatic alcohols containing from 8 to 22 carbon atoms, in either straight chain or branched configuration, with from 3 to 12 moles of ethylene oxide per mole of alcohol. Particularly preferred are the condensation products of alcohols having an alkyl group containing from about 9 to 15 carbon atoms with from about 4 to 8 moles of ethylene oxide per mole of alcohol.

Semi-polar nonionic surfactants include water-soluble amine oxides containing one alkyl moiety of from about 10 to 18 carbon atoms and two moieties selected from the group of alkyl and hydroxyalkyl moieties of from about 1 to about 3 carbon atoms; water-soluble phosphine oxides containing one alkyl moiety of about 10 to 18 carbon atoms and two moieties selected from the group consisting of alkyl groups and hydroxyalkyl groups containing from about 1 to 3 carbon atoms; and water-soluble sulphoxides containing one alkyl moiety of from about 10 to 18 carbon atoms and a moiety selected from the group consisting of alkyl and hydroxyalkyl moieties of from about 1 to 3 carbon atoms.

Ampholytic surfactants include derivatives of aliphatic or aliphatic derivatives of heterocyclic secondary and tertiary amines in which the aliphatic moiety can be straight chain or branched and wherein one of the aliphatic substituents contains from about 8 to 18 carbon atoms and at least one aliphatic substituent contains an anionic water-solubilizing group.

Zwitterionic surfactants include derivatives of aliphatic, quaternary, ammonium, phosphonium, and sulphonium compounds in which one of the aliphatic substituents contains from about 8 to 18 carbon atoms.

The level of detergent surfactant that can be employed is from 0% to about 50%, preferably from about 1% to about 30% and most preferably from about 10% to about 25% by weight of the total composition.

In addition to detergent surfactants, detergency builders can be employed in the bleaching compositions. Water-soluble inorganic or organic electrolytes are suitable builders. The builder can also be water-insoluble calcium ion-exchange materials; non-limiting examples of suitable water-soluble, inorganic detergent builders include : alkali metal carbonates, borates, phosphates, bicarbonates and silicates. Specific examples of such salts include sodium and potassium tetraborates, bicarbonates, carbonates, orthophosphates, pyrophosphates, tripolyphosphates and metaphosphates.

Examples of suitable organic alkaline detergency builders include:
(1) water-soluble amino carboxylates and amino polyacetates, for example, nitrilotriacetates, glycinates, ethylene diamine tetraacetates, N-(2-hydroxyethyl)nitrilodiacetates and diethylene triamine pentaacetates;
(2) water-soluble salts of phytic acid, for example, sodium and potassium phytates;
(3) water-soluble polyphosphonates, including sodium, potassium, and lithium salts of ethane-1-hydroxy-1, 1-diphosphonic acid; sodium, potassium, and lithium salts of ethylene diphosphonic acid; and the like;
(4) water-soluble polycarboxylates such as the salts ot lactic acid, succinic acid, malonic acid, maleic acid, citric acid, carboxymethyloxy succinic acid, 2-oxa-1,1,3-propane tricarboxylic acid, 1,1,2,2-ethane tetracarboxylic acid, mellitic acid and pyromellitic acid; and
(5) water-soluble polyacetals as disclosed in US Patents 4,144,266 and 4,246,495.

Another type of detergency builder material useful in the present compositions comprises a water-soluble material capable of forming a water-insoluble reaction product with water hardness cations, preferably in combination with a crystallization seed which is capable of providing growth sites for said reaction product. Such "seeded builder" compositions are fully disclosed in British Patent Specification No. 1,424,406.

A further class of detergency builder materials useful in the present invention are insoluble sodium aluminosilicates, particularly those described in Belgian Patent 814,874. This patent discloses detergent compositions containing sodium aluminosilicates having the formula :

Na₂(AlO₂)_{z}(SiO₂)_{y}XH₂0

wherein z and y are integers equal to at least 6, the molar ratio of z to y is in the range of from 1.0:1 to about 0.5:1, and X is an integer from about 15 to about 264, said aluminosilicates having a calcium ion exchange capacity of at least 200 milligrams equivalent/gram and a calcium ion exchange rate of at least about 2 grains/gallon/minute/gram. A preferred material is Zeolite A which is:

Na₁₂(SiO₂Al0₂)₁₂27H₂0

The level of detergency builder of the bleaching compositions is from 0% to about 70%, preferably from about 10% to about 60% and most preferably from about 20% to about 60%.

Buffering agents can be utilized to maintain the desired alkaline pH of the bleaching solutions. Buffering agents include, but are not limited to, many of the detergency builder compounds disclosed hereinbefore. Buffering agents suitable for use herein are those well known in the detergency art.

Preferred optional ingredients include suds modifiers, particularly those of suds-suppressing types, exemplified by silicones, and silica-silicone mixtures.

US Patents 3,933,672 and 4,136,045 disclose silicone suds-controlling agents.

The silicone suds-suppressing agent is advantageously releasably incorporated in a water-soluble or water-dispersible, substantially non-surface-active detergent-impermeable carrier.

Particularly useful suds suppressors are the self-emulsifying silicone suds suppressors described in US Patent 4,073,118. An example of such a compound is DB-544, commercially available from Dow Corning, which is a siloxane/glycol copolymer.

Suds modifiers as described above are used at levels of up to approximately 2%, preferably from about 0.1 to about 1.5% by weight of the surfactant.

Microcrystalline waxes having a melting point in the range of from 35°C-115°C and a saponification value of less than 100 represent additional examples of preferred suds control components for use in the subject compositions, and are described in detail in US Patent 4,056,481. The microcrystalline waxes are substantially water-insoluble, but are water-dispersible in the presence of organic surfactants. Preferred microcrystalline waxes have a melting point from about 65°C to 100°C, a molecular weight in the range from 400-1,000; and a penetration value of at least 6, measured at 77°F by ASTM-D1321. Suitable examples of the above waxes include: microcrystalline and oxidized microcrystalline petroleum waxes; Fischer-Tropsch and oxidized Fischer-Tropsch waxes; ozokerite; ceresin; montan wax; beeswax; candelilla; and carnauba wax.

Alkyl phosphate esters represent an additional preferred suds control agent for use herein. These preferred phosphate esters are predominantly monostearyl phosphate which, in addition thereto, can contain di- and tri-stearyl pbosphates and monooleyl phosphate, which can contain di- and tri-oleyl phosphate.

Other suds control agents useful in the practice of the invention are the soap or the soap and nonionic mixtures as disclosed in US Patents 2,954,347 and 2,954,348.

### EXAMPLE I

### Preparation of N-octyl succinimide

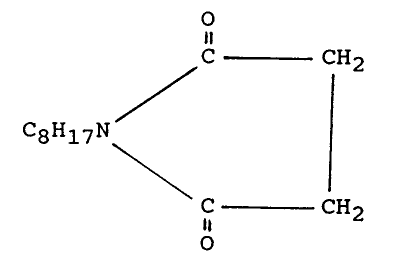

Succinic anhydride (10 g; 0.1 M) was dissolved with heating in ethanol-free chloroform (50 ml). A solution of n-octylamine (12.9 g; 0.1 M) in chloroform (20 ml) was added dropwise over 30 minutes and the mixture was stirred at room temperature for 18 hours. The resultant white precipitate was filtered, washed with chloroform (30 ml) and dried to give 4-octylamino-4-oxobutyric acid (19.84 g; 87%).

4-Octylamino-4-oxobutyric acid (5 g; 0.021 M) was heated in an oil bath at 120°C for 1 hour. During this time, water was distilled off and after 1 hour ceased, to leave an oil (4.4 g; 99%). This oil was decolourized by dissolving in ethanol (30 ml) and boiling the solution with activated charcoal to give a decolourized N-octylsuccinimide.

### EXAMPLE II

### Preparation of N-octylglutarimide

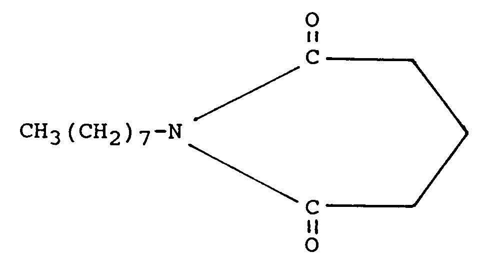

In a similar fashion as Example I, N-octylamine was reacted with glutaric anhydride in chloroform to give 5-octylamino-5-oxopentanoic acid (86% yield).

This material was heated at 250°C/0.8 mm Hg in a Kugelruhr distillation apparatus to produce N-octylglutarimide (81% yield).

### EXAMPLE III

### Preparation of trimethylammonium propyl glutarimide methosulphate

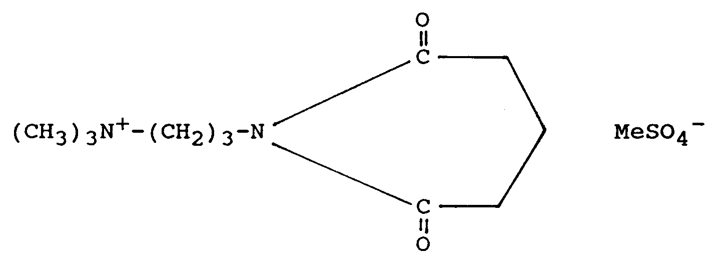

3-Dimethylamino propylamine (10.2 g; 0.1 M) was dissolved in anhydrous chloroform (200 ml) containing glutaric anhydride (11.4 g; 0.1 M) and the solution was stirred at room temperature for 1 hour. This mixture was then heated under reflux for 3 hours, after which the chloroform was removed under reduced pressure to yield an oil (21 g; 97%).

The oil (3-N,N-dimethyl aminopropyl glutaramide) - (1 g; 4.6 x 10⁻³ M) - was heated to 250°C at reduced pressure (0.8 mm Hg) in a Kugelruhr apparatus and an oil was collected (0.6 g; 66%). This oil was dissolved in acetonitrile (20 ml) containing dimethyl sulphate (0.38 g; 3 x 10⁻³ M) and the mixture was heated under reflux for 2 hours. The solvent was removed under reduced pressure and acetone (AR 10 ml) was added and the solution was sonicated for 0.5 hour and then cooled. A white solid trimethylammonium propyl glutarimide methosulphate crystallized out of solution, which was removed by filtration and dried in vacuo (0.7 g; 72%).

### EXAMPLE IV

### Preparation of trimethylammonium propyladipimide methosulphate

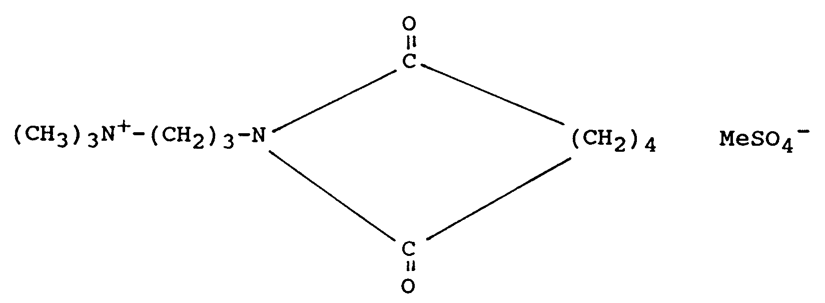

Adipic anhydride was prepared by heating adipic acid with acetic anhydride at 130°C. Performing the same series of reactions as in Example III with 3-dimethylaminopropyl amine gave rise to the corresponding adipamide : 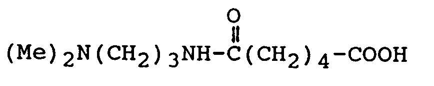
Heating of this compound at 250°C/0.6 mm Hg in the Kugelruhr distillation apparatus resulted in the corresponding cyclic imide, N,N-dimethylamino propyl adipimide. This amide was quaternized, using dimethyl sulphate to give the salt, trimethylammonium propyl adipimide methosulphate (TMA-PAI).

### EXAMPLE V

Bleaching tests were carried out in a 30 minutes' isothermal wash with bleach solutions containing 8 mMol/l of H₂O₂ and 1 mMol/l of TMA-PAI (molar ratio 8 : 1) at a temperature of 40°C on tea-stained test cloths.

The bleaching performances were determined by measuring the reflectance of the test cloths before and after the wash in an Elrepho reflectometer apparatus.

Similar good performance results were obtained when the bleaching tests were repeated, using 5 g/l of a conventional detergent base powder with sodium perborate monohydrate and TMA-PAI at the same molar concentrations.

The results were :
at pH 9.5 ΔR460* = 22.2
at pH 11 ΔR460* = 17.5

## Claims

1. A peroxyacid bleach precursor compound having the formula : 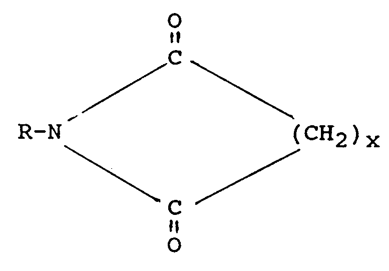 wherein R is an alkyl, aryl or alkaryl group containing from 1 to 10 carbon atoms, or a quaternary ammonium-substituted alkyl, aryl or alkaryl group containing 1 to 10 carbon atoms; and x is an integer from 2-6.

2. A precursor compound according to Claim 1, characterized in that R is an alkyl group containing 1 to 4 carbon atoms, or a phenyl (optionally substituted) group, or a trimethyl ammonium C₂-C₄-alkylene or phenylene group.

3. A precursor compound according to Claim 1 or 2, characterized in that x is from 2 to 4.

4. A precursor compound according to Claim 3, characterized in that R is a trimethyl ammonium alkylene or phenylene group.

5. A bleaching-detergent composition comprising a peroxide compound capable of yielding hydrogen peroxide in aqueous solution and a peroxyacid bleach precursor according to any one of Claims 1-4.

6. A composition according to Claim 5, characterized in that said peroxide compound and said bleach precursor are present in a molar ratio of from 2:1 to 20:1.

7. A composition according to Claim 6, characterized in that said molar ratio is from 5:1 to 12:1.

8. A composition according to Claim 5, 6 or 7, characterized in that it further comprises a detergent surfactant and a detergency builder.

9. A composition according to any one of Claims 5-8, characterized in that it has a pH of from 8.5 to 11.0.

## Patentansprüche

1. Vorstufenverbindung für ein Peroxysäurebleichmittel, die die Formel aufweist: 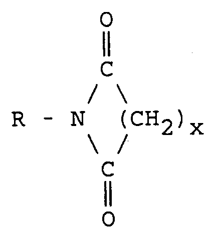 worin R eine Alkyl-, Aryl- oder Alkarylgruppe, die 1 bis 10 Kohlenstoffatome enthält oder eine quaternäre Ammonium-substituierte Alkyl-, Aryl- oder Alkarylgruppe, die 1 bis 10 Kohlenstoffatome enthält, bedeutet und x eine ganze Zahl von 2 bis 6 ist.

2. Vorstufenverbindung nach Anspruch 1, dadurch gekennzeichnet, daß R eine Alkylgruppe, die 1 bis 4 Kohlenstoffatome enthält oder eine Phenylgruppe (gegebenenfalls substituiert) oder eine Trimethylammonium-C₂-C₄-alkylen- oder -phenylengruppe bedeutet.

3. Vorstufenverbindung nach Anspruch 1 oder 2, dadurch gekennzeichnet, daß x 2 bis 4 ist.

4. Vorstufenverbindung nach Anspruch 3, dadurch gekennzeichnet, daß R eine Trimethylammoniumalkylen- oder -phenylengruppe ist.

5. Bleichendes Waschmittel, umfassend eine Peroxidverbindung, die in der Lage ist, Wasserstoffperoxid in wässeriger Lösung entstehen zu lassen und eine Vorstufe nach einem der Ansprüche 1 bis 4 für ein Peroxysäurebleichmittel.

6. Mittel nach Anspruch 5, dadurch gekennzeichnet, daß die Peroxidverbindung und die Bleichmittelvorstufe in einem Molverhältnis von 2:1 bis 20:1 vorliegen.

7. Mittel nach Anspruch 6, dadurch gekennzeichnet, daß das Molverhältnis 5:1 bis 12:1 beträgt.

8. Mittel nach Anspruch 5, 6 oder 7, dadurch gekennzeichnet, daß es außerdem ein Waschmitteltensid und einen Waschmittelbuilder umfaßt.

9. Mittel nach einem der Ansprüche 5 bis 8, dadurch gekennzeichnet, daß es einen pH-Wert von 8,5 bis 11,0 aufweist.

## Revendications

1. Un composé précurseur de blanchiment au peroxyacide présentant la formule : 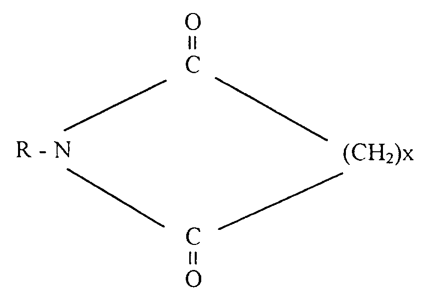 où R est un groupe alkyle, aryle ou alkaryle contenant 1 à 10 atomes de carbone, ou un groupe alkyle, aryle ou alkaryle substitué par un ammonium quaternaire et contenant 1 à 10 atomes de carbone ; et où x est un entier compris entre 2 et 6.

2. Un composé précurseur selon la Revendication 1, caractérisé en ce que R est un groupe alkyle contenant 1 à 4 atomes de carbones, ou un groupe phényle (optionnellement substitué), ou un groupe alkylène en C₂ à C₄ ou phénylène de triméthylammonium.

3. Un composé précurseur selon la Revendication 1 ou 2, caractérisé en ce que x est compris entre 2 et 4.

4. Un composé précurseur selon la Revendication 3, caractérisé en ce que R est un groupe alkylène ou phénylène de triméthylammonium.

5. Une composition détergente de blanchiment comprenant un composé peroxyde capable de donner un peroxyde d'hydrogène en solution aqueuse et un précurseur de blanchiment au peroxyacide en accord avec l'une quelconque des Revendications 1 à 4.

6. Une composition selon la Revendication 5, caractérisée en ce que ledit composé peroxyde et ledit précurseur de blanchiment sont présents dans un rapport molaire compris entre 2:1 et 20:1.

7. Une composition selon la Revendication 6, caractérisée en ce que le rapport molaire est compris entre 5:1 et 12:1.

8. Une composition selon la Revendication 5, 6 ou 7, caractérisée en ce qu'elle comprend en outre un agent tensioactif détergent et un édificateur de détergence.

9. Une composition selon l'une quelconque des Revendications 5 à 8, caractérisée en ce qu'elle présente un pH compris entre 8,5 et 11,0.
